# EUROPEAN PATENT APPLICATION

(11) **EP 4 821 480 A1**
(43) Date of publication of application: **14.10.2026**
(21) Application number: 25168953.5
(22) Date of filing: 07.04.2025
(51) Int. Cl.: A61K 8/64, A61Q 19/02, A61Q 19/08

(54) **USE OF S-ACETYL-GLUTATHIONE AND CRYSTALLINE FORMS THEREOF IN COSMETIC FORMULATIONS FOR ANTIOXIDANT, ANTI-AGEING AND SKIN WHITENING APPLICATIONS**

(71) Applicant: Lesaffre et Compagnie, 75001 Paris (FR)
(72) Inventor: BELLEMERE, Gaelle, 28300 Champhol (FR); MANIERE, Audrey, 92160 Antony (FR); VANDENHAUTE, Elodie, 62440 Harnes (FR)
(74) Representative: Bianchetti & Minoja with Trevisan & Cuonzo IPS SRL

(57) **Abstract**

The present invention provides cosmetic compositions comprising S-acetyl-glutathione as an active ingredient for antioxidant protection, anti-ageing benefits, and skin-whitening effects. The invention also provides methods of using such compositions for preventing oxidative damage, protecting against iron-induced skin ageing, and reducing hyperpigmentation.

## Description

### Field of invention

The present invention relates to cosmetic formulations comprising a S-acetyl glutathione (SAG) or a crystalline form thereof.

### Background to the invention

Glutathione (GSH) is a compound which, in its reduced form, constitutes an excellent antioxidant and therefore a defence against the damage caused by free radicals to higher organisms. S-acetyl-glutathione (SAG) is a synthetic derivative thereof which protects GSH against oxidation, and simultaneously releases it easily by hydrolysis.

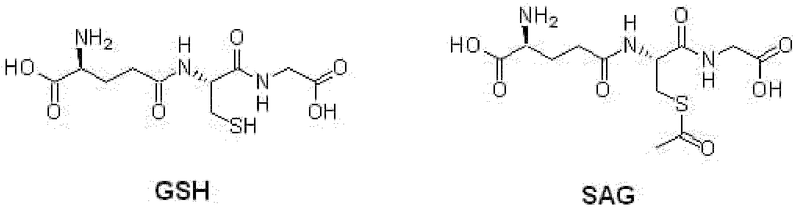

GSH or γ-L-glutamyl-L-cysteinylglycine is a tripeptide consisting of glutamic acid, cysteine and glycine, characterised by an atypical peptide bond, namely the bond that binds the nitrogen of cysteine to the carboxyl in y glutamic acid. It is the main thiol compound with the lowest molecular weight present in both animal and plant cells (about 95% of the total). Its function is to maintain in the reduced state the -SH groups of many enzymes and proteins whose oxidation (with the formation of S-S intra- and intermolecular disulphide bridges) leads, in most cases, to inactivation or loss of the biological function of the enzyme or protein.

GSH is considered to be one of the most important intracellular antioxidants produced naturally by the human body. For this reason, GSH and derivatives thereof have extensively been used as active ingredients in pharmaceutical and nutraceutical compositions for enteral or parenteral administration.

Topical uses of glutathione derivatives have also been disclosed. US 3,948,569 discloses the use of S-substituted alkyl and alkenyl derivatives of glutathione for scalp and hair applications and to combat excessive sebum secretion. US 5,516,507 discloses glutathione mono-alkyl esters for topical treatment of cutaneous aging. US 2004/0147452 proposes the use of non-amphoteric N-acyl glutathione derivatives for topical application.

US 20140045767 discloses compositions comprising S-acyl glutathiones derivatives, in particular S-palmitoyl glutathione, to prevent and to treat skin aging.

EP 3 066 111 B1 discloses the preparation of a crystalline form of S-acetyl glutathione, hereinafter referred to as SAG Form A, characterized by advantageous properties in terms of stability, dissolution rate and powder density.

### Description of the invention

It has now been found that S-acetyl-glutathione (SAG) plays a crucial role in protecting skin from iron-induced oxidative stress, which is particularly relevant for hormone-depleted skin during menopause. Moreover, SAG has proven to be effective also as anti-stain and skin whitening agent.

S-acetyl-glutathione in cosmetic applications offers enhanced stability, increased cellular uptake, and multi-functional benefits, including antioxidant protection, anti-ageing effects, and skin whitening. Unlike glutathione, SAG remains stable in various cosmetic formulations, ensuring long-term efficacy. Its ability to easily permeate cell membranes increases its potency as an active ingredient in skin-care products.

SAG acts as a powerful intracellular antioxidant, reducing oxidative stress within skin cells. By effectively crossing the plasma membrane, SAG enhances mitochondrial protection, helping to maintain cellular health and functionality.

SAG helps prevent iron oxidation in the skin, thereby reducing oxidative damage that contributes to premature ageing, particularly in post-menopausal skin.

SAG exhibits superior efficacy in reducing skin pigmentation and improving overall skin tone compared to conventional glutathione.

The present invention accordingly provides cosmetic compositions comprising S-acetyl-glutathione as an active ingredient for antioxidant protection, anti-ageing benefits, and skin-whitening effects. The invention also provides methods of using such compositions for preventing oxidative damage, protecting against iron-induced skin ageing, and reducing hyperpigmentation.

The proposed formulations and methods of use provide innovative solutions for improving skin health and appearance.

### Detailed Description of the Invention

S-acetyl glutathione or its crystalline form A are preferably formulated in forms suited for the topical application to the skin tissue.

Examples of suitable formulations include lotions containing oils and/or alcohols and emollients vegetable oils, hydrocarbon oils and waxes, silicone oils, animal or marine fats or oils, glyceride derivatives, fatty acids or fatty acid esters, or alcohols or alcohol ethers, lecithin, lanolin and derivatives, polyhydric alcohols or esters, wax esters, sterols, phospholipids and the like, emulsifiers (nonionic, cationic or anionic). Other suitable formulations are creams, ointments, gels, sticks, powders, masks, soaps, serums, wipes, emulsions.

The crystalline form A of S-acetyl glutathione is preferred. Said form A, characterized by an X-ray powder diffraction spectrum, obtained using the α1 and α2 radiations of copper at 1.54060Å and 1.54439Å respectively, having characteristic peaks, expressed in degrees 2-theta [°], at 5.2, 10.3, 15.4, 18.6, 19.7, 35.3, 36.3 ± 0.2, is obtainable by crystallizing SAG with mixtures of solvents such as water-acetone, water-ethanol and water-methanol, preferably water-acetone, as disclosed in EP 3 066 111 B1.

The concentration by weight S-acetyl glutathione with respect to the total of the composition will typically range between about 0.01% to about 20%, preferably, from about 0.1% to about 5%. If desired, the composition of the invention may include other cosmetically active ingredients, in addition to S-acetyl glutathione.

The topical compositions of the invention may contain additional ingredients commonly found in skin care compositions and cosmetics, such as, for example, emollients, skin conditioning agents, emulsifying agents, surfactants, humectants, preservatives, antioxidants, thickeners and gelling agents, soothing and anti-inflammatory agents, perfumes, etc.

Examples of emollients, typically present in amounts ranging from about 0.1% to about 5% by weight of the total composition, include vegetable oils such as Jojoba oil, sweet almond oil, argan oil, coconut oil, rosehip oil; butters such as Shea butter, cocoa butter, mango butter; esters such as isopropyl myristate, caprylic/capric triglyceride, silicones such as dimethicone, cyclopentasiloxane.

Examples of humectants include glycerin, hyaluronic acid, sorbitol, propylene glycol, butylene glycol, sodium PCA.

Examples of emulsifiers, typically present in amounts from about 1% to about 15% by weight, include cetearyl alcohol, glyceryl stearate, lecithin, polyglyceryl-3 methylglucose distearate, Ceteareth-20.

Examples of surfactants include lauryl glucoside, cocamidopropyl betaine, sodium cocoyl isethionate, sodium lauryl sulfate.

Examples of thickeners and gelling agents, typically present in amounts from about 0.1% to about 10% by weight, include carbomers, xanthan gum, hydroxyethylcellulose, Guar hydroxypropyltrimonium chloride.

Examples of preservatives, typically present in amounts from about 0.001% to about 1.5% by weight, include phenoxyethanol, sodium benzoate, potassium sorbate, benzyl alcohol, ethylhexylglycerin.

Examples of antioxidants, typically present in an amount ranging from about 0.01% to about 1% by weight of the composition, include vitamin E, ascorbic acid, ferulic acid, coenzyme Q10. Examples of soothing and anti-inflammatory agents, typically present in amounts from about 1% to about 15% by weight, include bisabolol, allantoin, Chamomile extract, Aloe vera. Examples of sunscreen agents typically present in amounts from about 0,1% to about 5 % by weight, include zinc oxide, titanium dioxide, avobenzone, octocrylene, homosalate.

The utility of SAG, and particularly of SAG form A, as cosmetically active ingredient has been evaluated in a model of oxidative stress in human primary fibroblasts. The effect of SAG has been studied by measuring the protection of mitochondrial integrity and/or the prevention of mitochondria destruction induced by hydrogen peroxide or menadione. SAG promotes mitochondrial health.

The following examples further illustrate the invention.

### Example 1

### Preparation of SAG in crystalline form A

5 g of crude SAG is placed under stirring and heated to 75°C in 40 mL of demineralised water. The reaction mass is heated to 75°C-80°C. After dissolution, the solution is immediately cooled to a temperature of under 55°C, preferably between 45 and 55°C. Cooling continues until crystallisation begins. Stirring is minimized and the solution is cooled to 20-25°C, at which temperature it is left under stirring for 2-12 h until precipitation is complete. Subsequently, again with minimal stirring, 40 mL of acetone is added in about 30-50 min. The addition is slow to prevent the formation of even a few crystals of polymorph B. The resulting suspension is then brought to 5°C ± 2°C and maintained under slow stirring (60-120 rpm) for about 1 h. At the end of that time the reaction mass is filtered to obtain a white solid, which is washed with anhydrous acetone (2 x 10 mL). 8.4 g of wet solid is thus obtained, which is left to dry at 50°C, 5 mbar of residual vacuum for 14-18 h. 4.3 g (86%) of white crystalline solid corresponding to crystalline form A is obtained after drying.

The analytical profile of the product thus obtained is:
Assay value: 98.6% (as is)
Impurities: Total: 1.0%; Single known impurities: GSH (0.1%), GSSG (2.2%);
Water 1.4%
Residual acetone: < 500 ppm
Residual acetic acid: 0.4%
Apparent density: 0.15-0.25 g/mL

The product thus obtained presents X-ray powder diffraction spectrum, obtained with α1 (λ =1.54060Å) and α2 (λ =1.54439Å) copper radiation, having characteristic peaks, expressed in degrees 2-theta [°], at 5.2, 10.3, 15.4, 18.6, 19.7, 35.3, 36.3 ± 0.2.

In the XRD diffractogram, an additional group of characterizing diffraction peaks, expressed in degrees 2-theta [°], is represented by those at 20.4, 21.1, 25.1, 25.7, 27.0, 27.6, 27.9, 32.7 ± 0.2.

Crystalline form A is characterized by an IR spectrum, obtained with a potassium bromide matrix, having characteristic absorption bands at 3344, 1726, 1687 and 1663 cm-1 (inter alia). Crystalline form A is also characterized by a DSC pattern, obtained with a heating rate of 10.00°C/min, having an endothermic peak between 190°C and 210°C, connected to the decomposition of the compound.

### Example 2

### Effect of SAG on mitochondrial health in human primary fibroblasts subjected to oxidative stress

Mitochondrial network from fibroblast, whether stressed with H₂O₂/menadione or unstressed, were analysed using MitoTracker Red CMXROS staining. MitoTracker Red CMXROS is a fluorescent dye specific to label mitochondria (Agnello, M., et al., A.M. (2008), A method for measuring mitochondrial mass and activity, Cytotechnology 56, 145-149; Cottet-Rousselle, C., et al. (2011), Cytometric assessment of mitochondria using fluorescent probes, Cytom Part A 79A, 405-425.

Preliminarly, the cytotoxicity of the S-acetyl glutathion and glutathion GSH was assessed. Primary human fibroblasts were treated with active ingredients at either 1% - 0,5% - 0,2% - 0,1% or 0,05%. Triton 0,1% was used as positive control. The cell toxicity was measured after 3 days of treatment using WST-1 reagent. S-acetyl glutathion at 1%, glutathion GSH at 1% seemed to induce fibroblasts mortality. The concentrations of S-acetyl glutathion chosen for this study were 0,2%, 0,1% and 0,05%. The concentrations of glutathion chosen for the rest of this study were 0,176%, 0,088% and 0,044%. Fibroblasts were treated for 24 hours with active ingredient. Then, fibroblasts were stained by 2 fluorescent markers : nuclear stain (blue), MitoTracker CMXROS red detection reagent (orange). Experiment was performed in triplicate for each condition. Pictures were taken with a CellInsight NXT Platform (10X) (ImageJ software) equipped with a 386 channel (blue), a 549nm channel (orange). A custom python algorithm was used to measure the mitochondria network area or total intensity per cells. Statistical analyses were performed using T test: comparison with Control (*p-value < 0.1, *p-value < 0.05, **p-value < 0.01, ***p-value < 0.001).

The study showed that S-acetyl glutathion at 0,04%, 0,176%, 0,2% and 0,1% counterbalanced the strong reduction of MitoTracker area and total intensity induced by H₂O₂ or menadione treatment. S-acetyl glutathion proves therefore to be able to prevent accumulation of defective and dysfunctional mitochondria in cells, responsible of skin functions impairment.

## Claims

1. A cosmetic composition comprising S-acetyl-glutathione or a crystalline form thereof as an active ingredient in admixture with suitable excipients, possibly in combination with other cosmetically active ingredients.

2. A composition according to claim 1 for topical application.

3. A composition according to claim 2 in form of creams, ointments, gels, sticks, powders, masks, soaps, serums, wipes, emulsions.

4. A composition according to any one of claims 1-3 wherein the crystalline form of glutathione is form A **characterized by** an X-ray powder diffraction spectrum, obtained using the α1 and α2 radiations of copper at 1.54060Å and 1.54439Å respectively, having characteristic peaks, expressed in degrees 2-theta [°], at 5.2, 10.3, 15.4, 18.6, 19.7, 35.3, 36.3 ± 0.2.

5. A composition according to any one of claims 1-4 wherein the excipients are selected from emollients, skin conditioning agents, emulsifying agents, surfactants, humectants, preservatives, antioxidants, thickeners and gelling agents, soothing and anti-inflammatory agents, perfumes.

6. Use of S-acetyl-glutathione or a crystalline form thereof for reducing oxidative stress and/or for preventing iron-induced skin ageing, and/or for enhancing mitochondrial function and/or cellular protection against oxidative damage and/or as anti-stain and skin whitening agent.

7. A method of reducing skin ageing, particularly in hormone-depleted skin, by topically applying a composition of claims 1-5.
